(19) Europäisches Patentamt
European Patent Office
Office européen des brevets

(11) Numéro de publication : **0 326 456 B1**

(12) # FASCICULE DE BREVET EUROPEEN

(45) Date de publication du fascicule du brevet : **11.11.92 Bulletin 92/46**

(51) Int. Cl.$^5$ : **C07C 50/24,** C07C 46/00, C07C 46/06

(21) Numéro de dépôt : **89400119.7**

(22) Date de dépôt : **16.01.89**

(54) **Procédé de préparation de chloranil.**

(30) Priorité : **27.01.88 FR 8800910**

(43) Date de publication de la demande : **02.08.89 Bulletin 89/31**

(45) Mention de la délivrance du brevet : **11.11.92 Bulletin 92/46**

(84) Etats contractants désignés : **AT BE CH DE ES FR GB GR IT LI LU NL SE**

(56) Documents cités :
**DE-A- 2 645 114**
**DE-A- 2 933 119**
**ANGEW. CHEM. INT. ED. ENGL., vol. 15, no. 10, 1976, page 608; H. LÜBBECKE et al.: "Nonpollutative chlorination andchlorinating oxidation of phenols"**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière Route de Ternay**
**F-69360 Communay (FR)**
Inventeur : **Jouve, Isabelle**
**30, rue Léon Fabre**
**F-69100 Villeurbanne (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de chloranil à partir d'hydroquinones ou de quinones éventuellement en partie chlorées pouvant être mises en oeuvre dans un appareillage le plus simple possible.

Le chloranil est un intermédiaire de synthèse fort connu et utilisé dans la synthèse de matières colorantes. Les procédés de synthèse décrits dans la littérature d'un tel composé sont nombreux, les rendements annoncés sont pour certains procédés excellents, de l'ordre de 95 % et même supérieurs.

Il a par exemple été décrit, il y a déjà très longtemps par R. Schuloff, R. Pollak dans Chemiker-Zeitung 56, p. 569 (1932) la chloration de la quinone en présence de solutions concentrées d'acide chlorhydrique. La chloration est effectuée à haute température pendant des durées de réaction très longues. A cause de la température élevée et des quantités importantes de chlore qui sont utilisées, il y a d'une part sublimation d'une partie du chloranil qu'il faut condenser pour le récupérer et d'autre part une forte pollution due au chlore excédentaire n'ayant pas réagi.

Il est aussi connu de nombreux procédés de chloration utilisant l'acide chlorhydrique concentré en présence de catalyseurs variés. Il est par exemple décrit dans le brevet allemand 2 645 114 un procédé de chloration oxydante de l'hydroquinone en présence de dichlorure de magnésium, dans la publication effectuée par Fischer et Henderson parue dans Synthesis 1985, (6.7), 641-3., l'oxydation est effectuée en présence de nitrate de cérium, dans la publication effectuée par Rettig et Latscha (Z. Naturforsch, 35 b, 399-400 (1980) la chloration est effectuée en présence de pentachlorure d'antimoine.

L'industrie a toujours cherché à éviter l'utilisation de catalyseurs de chloration dans tout procédé pouvant fonctionner en l'absence de ceux-ci.

Ainsi il a été proposé dans le brevet EP 220 135 un procédé de chloration oxydante d'hydroquinone ou de quinone en l'absence de tout catalyseur, le procédé étant caractérisé par l'utilisation d'une pression élevée de chlore comprise entre 3 et 40 bar. La concentration de l'hydroquinone dans l'acide chlorhydrique ne dépasse jamais 70 g/litre ce qui est faible pour un procédé industriel, en outre la pression utilisée exige un appareillage spécial.

Par ailleurs, selon DE-A-2 933 119, il est connu de préparer le bromanil par mise en contact d'hydroquinone avec une solution d'acide bromhydrique à 62 % et de peroxyde d'hydrogène.

Il est précisé dans ce document, que le peroxyde d'hydrogène est introduit à une température légèrement supérieure à la température ambiante (30°C) et que le chauffage est effectué ensuite aux alentours de 60°C, après l'introduction de tout le peroxyde d'hydrogène.

La préparation du chloranil à partir de sources phénoliques a été abandonnée par toute l'industrie chimique car ils forment au cours de la chloration oxydante des sous produits du type dioxine dont la toxicité est telle que leur présence doit être à tout prix évitée (décret allemand sur les substances dangeureuses de septembre 1986 § 9 (6)).

La présente invention a permis de résoudre les problèmes laissés par l'art antérieur, elle permet de s'affranchir de la présence de catalyseurs halogénométalliques, elle permet de travailler à la pression atmosphérique, elle permet en dernier de travailler avec de fortes concentrations en matières premières de l'ordre de 100 g par litre réactionnel.

L'invention est caractérisée par le fait qu'on réalise une chloration oxydante d'une quinone ou d'une hydroquinone éventuellement chlorée par un mélange acide chlorhydrique-péroxyde d'hydrogène, en l'absence de catalyseur de chloration caractérisée en ce qu'on introduit le péroxyde d'hydrogène à une température supérieure à 60°C.

On préfère introduire le péroxyde d'hydrogène à une température supérieure à 70°C.

Lors de l'introduction du péroxyde d'hydrogène au départ et à froid (brevet DE 2 645 114) il y a accumulation de péroxyde d'hydrogène dans le réacteur ce qui pose des problèmes de sécurité et, de plus, entraîne la formation de mousses importantes et des difficultés pour contrôler la réaction.

L'introduction du péroxyde d'hydrogène dans le milieu réactionnel à chaud est faite de préférence en continu pour éviter l'accumulation de ce dernier dans le réacteur.

La quinone éventuellement chlorée répond à la formule (I) suivante

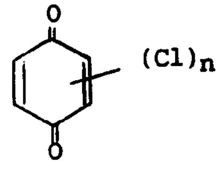

(I)

dans laquelle n est égal ou supérieur à 0 et égal ou inférieur à 3.

L'hydroquinone éventuellement chlorée répond à la formule (II) suivante

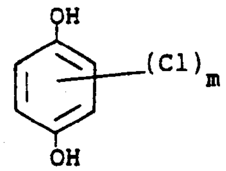

(II)

dans laquelle m est égal ou supérieur à 0 et égal ou inférieur à 4.

On préfère utiliser comme matière première l'hydroquinone non substituée car elle représente la matière première la moins onéreuse.

Pour une meilleure mise en oeuvre de l'invention il est avantageux d'utiliser un milieu réactionnel saturé en acide chlorhydrique, pour cela on préfère utiliser une solution aqueuse d'acide chlorhydrique ION ou contenant 30 à 37% en poids d'acide chlorhydrique. Pour saturer le milieu il est en outre avantageux d'introduire en continu de l'acide chlorhydrique gazeux.

La solution de péroxyde d'hydrogène utilisée est une solution aqueuse la plus concentrée possible. Une solution contenant 30 % en poids de péroxyde d'hydrogène est tout à fait conseillée.

On utilise au moins 5 moles de péroxyde d'hydrogène par mole d'hydroquinone et un excès de 20 % est préférentiellement mis en oeuvre.

La quinone ou l'hydroquinone est introduite dans le milieu réactionnel constitué d'acide chlorhydrique à une concentration de préférence inférieure à 130-140 g/litre. Une concentration de l'ordre de 100 g est tout à fait conseillée (à vérifier).

La température réactionnelle est supérieure à 60°C et de préférence supérieure à 70°C. Une température d'environ 90°C est avantageuse. La pression utilisée est la pression atmosphérique.

Les exemples suivants permettront de mieux comprendre l'invention. Ils ne sont donnés qu'à titre illustratif et ne doivent pas être considérés comme limitatifs de l'invention.

Les abréviations suivantes seront utilisées :

- HQ : hydroquinone
- $Cl_3BQ$ : trichlorobenzoquinone
- $Cl_4HQ$ : tétrachlorohydroquinone
- RR : rendement sur le produit introduit.

Essai comparatif selon le brevet DE 2 645 114

Dans un réacteur de 500 ml muni d'une agitation centrale à pales, d'un réfrigérant, d'une ampoule de coulée, d'un thermomètre, on charge 200 ml d'acide chlorhydrique à 35 % (2,28 M), 17,63 g de $MgCl_2$, $6H_2O$ (0,086 M), 2,82 g d'hydroquinone (0,0256 M).

Après avoir porté le mélange réactionnel à 0°, 15 ml d'eau oxygénée à 30 % (0,146 M) sont coulés en 15 minutes. Le mélange est ensuite porté à 100° en 1 h 30 ce qui provoque la formation de mousse montant dans le réfrigérant. Après deux heures de chauffage à 100°, le mélange est refroidi, puis le précipité est recueilli par filtration sur fritte. Le précipité est lavé par 3 x 20 ml d'eau et séché à l'étuve. Le précipité recueilli pèse 5,89 g soit pondéralement 93,6 % du chloranil attendu. Le dosage par chromatographie phase liquide de ce produit montre qu'il contient 49,2 % de chloranil et 44,8 % de tétrachlorobenzoquinone ce qui conduit respectivement à des RR de 46,2 % et de 41,7 %. Le produit brut contient également 6 % de chlorure de magnésium.

On réalise le même essai selon l'invention en introduisant le péroxyde d'hydrogène en 1 heure dans une solution d'acide chlorhydrique contenant l'hydroquinone maintenue à 80°C et en l'absence de catalyseur ($MgCl_2$).

| Essai | Concentration HQ initiale | Excès $H_2O_2$ | Coulée $H_2O_2$ | | Chauffage | | Rdt produit isolé | Composition | | |
|---|---|---|---|---|---|---|---|---|---|---|
| | | | durée | température | durée | température | | Chloranil | $Cl_3BQ$ | $Cl_4HQ$ |
| Comparatif | 14 g/l | 20 % | 15' | 0°C | 1 H 30<br>2 H | 0 à 100 °C<br>100°C | 93,9 % | 49,2 % | | 44,8 % |
| 1 | 15 g/l | 20 % | 50' | 80°C | 60' | 90°C | 92,8 % | 89,4 % | 5,3 % | 5 % |
| 2 | 18 g/l | 20 % | 20' | 80°C | Filtration à chaud à 80°C | | 82 % | 97,8 % | 0,8 % | |

Comparatif : mousse importante,

Essais 1 et 2 : absence de mousse.

## Revendications

1. Procédé de préparation de Chloranil par chloration oxydante d'une quinone ou d'une hydroquinone éventuellement chlorée par un mélange acide chlorhydrique-péroxyde d'hydrogène, en l'absence de catalyseur de chloration caractérisé en ce qu'on introduit le péroxyde d'hydrogène à une température supérieure à 60°C.

2. Procédé selon la revendication 1 caractérisé en ce que l'on introduit le péroxyde d'hydrogène à une température supérieure à 70°C.

3. Procédé selon la revendication 1 caractérisé en ce que la quinone éventuellement chlorée répond à la formule (I)

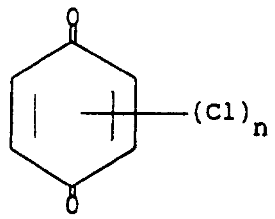

(I)

dans laquelle n est égal ou supérieur à 0 et égal ou inférieur à 3.

4. Procédé selon la revendication 1 caractérisé en ce que l'hydroquinone éventuellement chlorée répond à la formule (II)

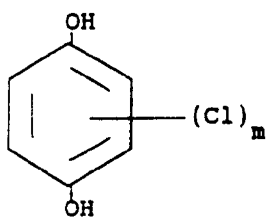

(II)

dans laquelle m est égal ou supérieur à 0 et égal ou inférieur à 4.

5. Procédé selon la revendication 4 caractérisé en ce que dans la formule (II) m est égal à 0.

6. Procédé selon la revendication 1 caractérisé en ce que l'acide chlorhydrique utilisé est une solution aqueuse contenant 30 à 37 % d'acide chlorhydrique.

7. Procédé selon la revendication 1 caractérisé en ce que le péroxyde d'hydrogène utilisé est une solution aqueuse contenant environ 30 % en poids de préroxyde d'hydrogène.

8. Procédé selon la revendication 1 caractérisé en ce que la température de réaction est comprise entre 80 et 90°C.

9. Procédé selon la revendication 1 caractérisé en ce que la pression utilisée est la pression atmosphérique.

## Patentansprüche

1. Verfahren zur Herstellung von Chloranil durch oxidierende Chlorierung eines gegebenenfalls chlorierten Chinons oder Hydrochinons mit einem Gemisch von Chlorwasserstoffsäure und Wasserstoffperoxid, in Abwesenheit eines Chlorierungskatalysators, dadurch gekennzeichnet, daß man das Wasserstoffperoxid

bei einer Temperatur über 60°C zugibt.

2. Verfahren nach Anspruch 1, dadurch gekennzeichnet, daß man das Wasserstoffperoxid bei einer Temperatur über 70°C zugibt.

3. Verfahren nach Anspruch 1, dadurch gekennzeichnet, daß das gegebenenfalls chlorierte Chinon der Formel (I)

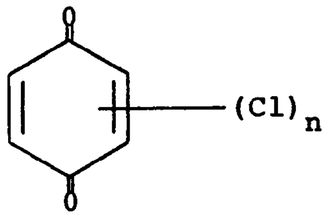

(I)

entspricht, in der n gleich oder größer 0 und gleich oder kleiner 3 ist.

4. Verfahren nach Anspruch 1, dadurch gekennzeichnet, daß das gegebenenfalls chlorierte Hydrochinon der Formel (II)

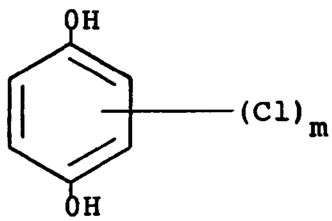

(II)

entspricht, in der m gleich oder größer 0 und gleich oder kleiner 4 ist.

5. Verfahren nach Anspruch 4, dadurch gekennzeichnet, daß in der Formel (II) m gleich 0 ist.

6. Verfahren nach Anspruch 1, dadurch gekennzeichnet, daß die Chlorwasserstoffsäure in wäßriger Lösung enthaltend 30 bis 37 % Chlorwasserstoffsäure eingesetzt wird.

7. Verfahren nach Anspruch 1, dadurch gekennzeichnet, daß das Wasserstoffperoxid in wäßriger Lösung eingesetzt wird, die etwa 30 Gew.% Wasserstoffperoxid enthält.

8. Verfahren nach Anspruch 1, dadurch gekennzeichnet, daß die Reaktionstemperatur zwischen 80 und 90°C liegt.

9. Verfahren nach Anspruch 1, dadurch gekennzeichnet, daß der verwendete Druck Atmosphärendruck ist.

## Claims

1. Process for the preparation of chloranil by oxychlorination of a quinone or of a hydroquinone, chlorinated, if desired, using a hydrochloric acid-hydrogen peroxide mixture, in the absence of any chlorination catalyst, characterised in that the hydrogen peroxide is introduced at a temperature above 60°C.

2. Process according to claim 1, characterised in that the hydrogen peroxide is introduced at a temperature above 70°C.

3. Process according to claim 1, characterised in that the quinone, chlorinated if desired, corresponds to the formula (I)

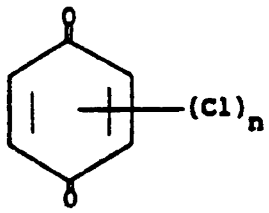

(I)

in which n is equal to or greater than 0 and equal to or smaller than 3.

4. Process according to claim 1, characterised in that the hydroquinone,, chlorinated if desired, corresponds to the formula (II)

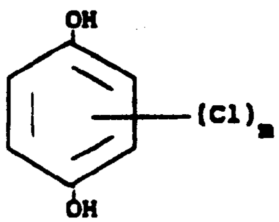

(II)

in which m is equal to or greater than 0 and equal to or smaller than 4.

5. Process according to claim 4, characterised in that in formula (II) m is equal to 0.

6. Process according to claim 1, characterised in that the hydrochloric arid employed is an aqueous solution containing 30 to 37% of hydrochloric acid.

7. Process according to claim 1, characterised in that the hydrogen peroxide employed is an aqueous solution containing approximately 30% by weight of hydrogen peroxide.

8. Process according to claim 1, characterised in that the reaction temperature is between 80 and 90°C.

9. Process according to claim 1, characterised in that the pressure employed is atmospheric pressure.